(19) Europäisches Patentamt
European Patent Office
Office européen des brevets

(11) Veröffentlichungsnummer: **0 016 731**
**B1**

(12) # EUROPÄISCHE PATENTSCHRIFT

(45) Veröffentlichungstag der Patentschrift: **16.02.83**

(21) Anmeldenummer: **80810090.3**

(22) Anmeldetag: **20.03.80**

(51) Int. Cl.³: **C 07 C127/19**, C 07 D211/16, C 07 D295/20, A 01 N 47/30, A 01 N 47/38

(54) **Meta-Cyanoalkoxy-Phenylharnstoffe mit herbizider Wirkung, deren Herstellung und sie enthaltende Mittel.**

(30) Priorität: **26.03.79 CH 2786/79**

(43) Veröffentlichungstag der Anmeldung: **01.10.80 Patentblatt 80/20**

(45) Bekanntmachung des Hinweises auf die Patenterteilung: **16.02.83 Patentblatt 83/07**

(84) Benannte Vertragsstaaten: **AT BE CH DE FR GB IT NL SE**

(56) Entgegenhaltungen: **DE A 2 044 735**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Patentabteilung Postfach**
**CH-4002 Basel (CH)**

(72) Erfinder: **Pissiotas, Georg, Dr.**
**Breslauerstrasse 8**
**D-7850 Lörrach (DE)**
Erfinder: **Rohr, Otto, Dr.**
**Kilbertweg 19**
**CH-4106 Therwil (CH)**

**EP 0 016 731 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Meta-Cyanoalkoxy-Phenylharnstoffe mit herbizider Wirkung, deren Herstellung und sie enthaltende Mittel

Die vorliegende Erfindung betrifft neue meta-Cyanoalkoxy-Phenylharnstoffe mit herbizider Wirkung, deren Herstellung, sie enthaltende Mittel sowie deren Verwendung zur Bekämpfung unerwünschten Pflanzenwachstums.

Die meta-Cyanoalkoxy-Phenylharnstoffe entsprechen der allgemeinen Formel I

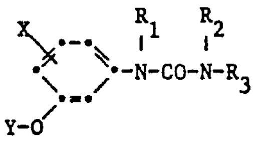

(I)

worin

X Wasserstoff oder Halogen,

Y eine Cyanoalkylgruppe

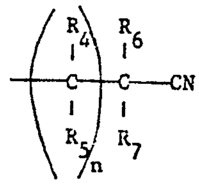

n eine Zahl 0, 1 oder 2,

$R_1$ Wasserstoff oder Niederalkyl,

$R_2$ Wasserstoff, Alkyl, Alkenyl, Alkinyl oder Alkoxy,

$R_3$ Wasserstoff, Alkyl oder Alkenyl oder $R_2$ und $R_3$ zusammen mit dem Stickstoffatom an das sie gebunden sind, auch einen 5-6 gliedrigen Heterocyclus, der als Ringglied noch ein Sauerstoff- oder Schwefelatom oder eine Iminogruppe enthalten kann,

$R_4$, $R_5$ und $R_7$ unabhängig voneinander je Wasserstoff oder Niederalkyl, und

$R_6$ Wasserstoff, Niederalkyl, Niederalkoxy oder Niederalkoxyalkyl bedeuten.

In der französischen Patentschrift Nr. 1.497.868 wird die herbizide Wirkung von N-(3-Chlor-4-methoxyphenyl)-N',N'-dimethylharnstoff beschrieben. Ferner sind aus der deutschen Offenlegungsschrift Nr. 2 044 735 Phenylharnstoffe bekannt, welche in para-Stellung zur Harnstoffgruppe eine Cyanoalkoxygruppe aufweisen und herbizid wirksam sind.

Es hat sich nun gezeigt, dass die erfindungsgemässen Verbindungen gegenüber den bekannten Verbindungen eine stärkere herbizide Wirkung aufweisen oder von manchen Kulturpflanzen weitaus besser vertragen werden.

Die Verbindungen der Formel I weisen ausgesprochen selektivherbizide Eigenschaften im allgemeinen auf und erweisen sich als besonders vorteilhaft zum Bekämpfen von Unkräutern in Kulturen von Nutzpflanzen, insbesondere in Kulturen von Soja, Baumwolle, Getreide, Mais und Zuckerrüben. Einzelne Verbindungen wirken selektiv in Raps.

Bei genügend grosser Aufwandmenge ist jedoch auch totalherbizide Wirkung vorhanden. Die Anwendung kann sowohl im Vorauflaufwie im Nachauflaufverfahren vorgenommen werden. Dabei können die Aufwandmengen in weiten Grenzen schwanken, z. B. zwischen 0,1 bis 10 kg Wirkstoff pro Hektare, vorzugsweise werden jedoch 0,5 bis 5 kg Wirkstoff pro Hektare eingesetzt.

Die erfindungemässen Mittel enthalten ausser dem Wirkstoff der Formel I einen geeigneten Träger und/oder andere Zuchlagstoffe. Geeignete Träger und Zuschlagstoffe können fest oder flüssig sein und entsprechen den in der Formulierungstechnik üblichen Stoffen wie z. B. natürlichen oder regenierten mineralischen Stoffen, Lösungs-, Verdünnungs-, Dispergier-, Emulgier-, Netz-, Haft-, Verdickungs-, Binde- und/oder Düngmitteln.

Zur Verwendung in herbiziden Mitteln kann die Verbindung der Formel I als Stäubemittel, Emulsionskonzentrat, als Granulat, Dispersion oder auch als Lösung oder Aufschlämmung in üblicher Formulierung verarbeitet werden.

Die Herstellung der Verbindungen der Formel I erfolgt nach an sich bekannten Methoden, z. B. durch Reaktion eines entsprechend substituierten Phenyls der Formel II

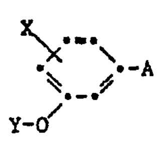

(II)

mit einem Amin der Formel III

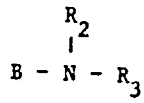

(III)

worin $R_2$, $R_3$, X und Y unter Formel I gegebene Bedeutung haben, während A und B Reste bedeuten, die unter Anlagerung oder Kondensation Harnstoffe bilden. Dabei stellt eine der beiden Gruppen A und B ein Amin dar, während die andere ein Urethan, ein Carbamoylhalogenid, eine Harnstoffgruppe, oder insbesondere den Isocyanatrest darstellt.

Ferner können ebenfalls meta-Hydroxyphenyl-harnstoffe der Formel IV

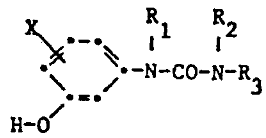

(IV)

mit einem Cyanoalkylhalogenid der Formel V

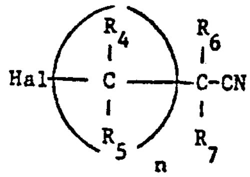

(V)

umgesetzt werden, in Gegenwart eines säurebindenden Mittels. In den Formeln IV und V haben X, n, $R_1$, $R_2$, $R_3$, $R_4$, $R_5$, $R_6$ und $R_7$ die unter der Formel I gegebene Bedeutung.

Es braucht nicht unbedingt ein Cyanoalkylhalogenid mit dem meta-Hydroxyphenylharnstoff umgesetzt werden, da die Cyanogruppe auch erst *in situ* in das Molekül eingeführt werden kann, zum Beispiel über die folgenden, hier schematisch wiedergegebenen Syntheseschritte :

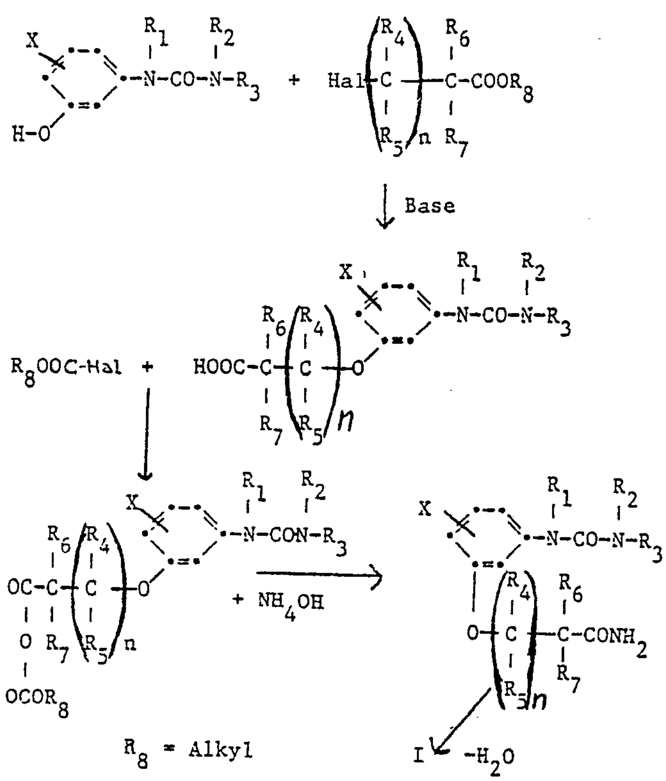


Darin wird ein meta-Hydroxyphenyl-Harnstoff mit einem Halogenalkancarbonsäureester kondensiert. Die Estergruppe wird dann verseift und die freie Carboxylgruppe anschliessend über das gemischte

Säureanhydrid mit Ammoniak in das Amid verwandelt und dieses mit einem wasserentziehenden Mittel zur Cyanoalkoxygruppe umgewandelt.

Diese Umsetzungen werden vorteilhafterweise in mit Wasser mischbaren organischen Lösungsmitteln wie Aceton, Butanon, Dimethylsulfoxid, Dimethylformamid usw. vorgenommen. Die Reaktionstemperaturen sind zwischen 0 °C und 150 °C gelegen, praktisch zwischen der Raumtemperatur und dem Siedepunkt des Reaktionsgemisches. Im allgemeinen wird unter Normaldruck gearbeitet, grössere Mengen können mit Vorteil auch in Druckgefässen hergestellt werden.

Die Verbindungen der Formel I sind für Warmblüter wenig giftig, ihre Handhabung bedarf keiner vorsorglicher Massnahmen. Sie sind in den üblichen organischen Lösungsmitteln relativ gut und in Wasser schlecht löslich. Sie können durch Zugeben von Wasser zur Reaktionslösung leicht ausgefällt werden. Ihre Formulierung als flüssige herbizide Mittel gelingt nur mit Hilfe besonderer Lösungsvermittler und/oder Dispergiermittel.

Die neuen Wirkstoffe der Formel I sind stabile Verbindungen, welche in üblichen organischen Lösungsmitteln, wie Alkoholen, Aethern, Ketonen, Dimethylformamid, Dimethylsulfoxyd etc. löslich sind.

Die Herstellung erfindungsgemässer Mittel erfolgt in an sich bekannter Weise durch inniges Vermischen und Vermahlen von Wirkstoffen der allgemeinen Formel I mit geeigneten Trägerstoffen und/oder Verteilungsmitteln, gegebenenfalls unter Zusatz von gegenüber den Wirkstoffen inerten Antischaum-, Netz-, Dispersions- und/oder Lösungsmitteln. Die Wirkstoffe können in den folgenden Aufarbeitungsformen vorliegen und angewendet werden :

Feste Aufarbeitungsformen : Stäubemittel, Streumittel, Granulate, Umhüllungsgranulate, Imprägniergranulate und Homogengranulate ;

In Wasser dispergierbare Wirkstoffkonzentrate : Spritzpulver (wettable powder), Pasten Emulsionen ; Emulsionskonzentrate ;

Flüssige Aufarbeitungsformen : Lösungen.

Die Wirkstoffkonzentrationen betragen in den erfindungsgemässen Mitteln 1 bis 80 Gewichtsprozent und können gegebenenfalls bei der Anwendung auch in geringen Konzentrationen wie etwa 0,05 bis 1 % vorliegen.

Den erfindungsgemässen Mitteln lassen sich andere biozide Wirkstoffe oder Mittel beimischen. So können die neuen Mittel ausser den genannten Verbindungen der allgemeinen Formel I z. B. Insektizide, Fungizide, Bakterizide, Fungistatika, Bakteriostatika, Nematozide oder weitere Herbizide zur Verbreiterung des Wirkungsspektrums enthalten.

Die folgenden Beispiele sollen die Herstellung der erfindungsgemässen Phenylharnstoffe der Formel I näher erläutern. Weitere in analoger Weise hergestellte Verbindungen sind in der sich dem Beispiel 2 anschliessenden Tabelle aufgeführt. Die Temperaturen sind in Celsiusgraden angegeben, Teile und Prozentangaben beziehen sich auf das Gewicht. In weiteren Beispielen wird die Aufbereitung der Wirkstoffe zu technisch verwendbaren Verabreichungsformen sowie Versuche zur Demonstration der herbiziden Wirkung beschrieben.

Beispiel 1

α-[m-(3,3-Dimethylureido) phenoxy]-propionitril :

Ein Gemisch von 9 g N-(m-Hydroxyphenyl)-N',N'-dimethylharnstoff, 7,5 g wasserfreiem Kaliumcarbonat, 6,7 g α-Brompropionitril und 100 ml Methyläthylketon wird 48 Stunden unter Rühren am Rückfluss erhitzt. Nach Abkühlen wird das Reaktionsgemisch filtriert und mit Methyläthylketon gewaschen. Nach dem Eindampfen im Vakuum erhält man 7,6 g Titelprodukt vom Schmelzpunkt 144-145°.

Beispiel 2

α-[m-(3,3-Dimethylureido)-phenoxy]-isobutyronitril :

a) Ein Gemisch von 180,2 g N-(m-Hydroxyphenyl)-N',N'-dimethylharnstoff, 140 g wasserfreiem Kaliumcarbonat, 197 g α-Brom-isobuttersäureäthylester und 1 000 ml Methyläthylketon wird 5 Tage lang unter Rühren zum Rückfluss erhitzt. Nach dem Abkühlen wird das Reaktionsgemisch filtriert und mit Methyläthylketon gewaschen, im Vakuum eingedampft und in Toluollösung mit Aktivkohle behandelt. Nach dem Abfiltrieren und Eindampfen im Vakuum erhält man 119 g α-[m-(3,3-Dimethylureido)-phenoxy]-isobuttersäureäthylester vom Schmelzpunkt 62-63°.

b) 73 g des unter a) beschriebenen Esters werden in 330 ml Methanol gelöst und mit 78 ml 3,5 N Natronlauge tropfenweise versetzt. Nach vierstündigem Rühren bei Raumtemperatur ist die Verseifung beendet. Nach dem Abdestillieren des Methanols wird das Reaktionsprodukt mit 2 N Salzsäure sauer gestellt wobei die freie Säure ausfällt. Man erhält 53,3 g α-[m-(3,3-Dimethylureido)-phenoxy]-isobuttersäure vom Schmelzpunkt 155-156°.

Man kann diese Säure auch wie folgt herstellen :

Ein Gemisch von 18 g N-(m-Hydroxyphenyl)-N',N'-dimethylharnstoff, 24 g pulverisiertem Natriumhydroxyd und 250 ml Aceton wird ca. 1 Stunde am Rückfluss erhitzt. Danach entfernt man das Wasserbad

und tropft unter Starkem Rühren eine Mischung von 36 g Chloroform und 50 ml Aceton dazu in solcher Weise, dass der Rückfluss während der Zugabe aufrecherhalten wird. Nach Beendigung dieser Zugabe wird das Reaktionsgemisch noch 4 Stunden unter Rückfluss weitergerührt. Anschliessend wird eingedampft, der Rückstand in 300 ml 2N Natronlauge aufgenommen, der unlösliche Teil mit Essigester extrahiert und die getrennte basisch-wässrige Lösung mit verdünnter Salzäure angesäuert, wobei die freie Säure ausfällt. Nach dem Abnutschen und Trocknen erhält man 20 g α-[m-(3,3-Dimethylureido)-phenoxy]-isobuttersäure vom Schmelzpunkt 196-197°.

c) 12 g der unter b) hergestellten Säure werden in 50 ml Dioxan vorgelegt und mit 10,7 ml Tri-n-butylamin vermischt, wobei ein Teil der Säure in Lösung geht. Die Mischung wird auf 10° gebracht und bei dieser Temperatur unter Rühren mit 4,3 ml Chlorameisensäureäthylester tropfenweise versetzt. Anschliessend wird 10 Minuten weitergerührt.

Nun wird ebenfalls bei 10° ein Ueberschuss an Ammoniak eingeleitet und 1 Stunde lang bei Raumtemperatur nachgerührt. Zum Schluss wird das Amid durch Zugabe von 50 ml Aether gefällt und abgenutscht. Die Ausbeute beträgt 10,5 g α-[m-(3,3-Dimethylureido)-phenoxy]-isobutersäureamid vom Schmelzpunkt 155-6 °C.

d) Ein Gemisch von 14 g des unter c) beschriebenen Amides, 29,4 g Triphenylphosphin, 10,8 ml Tetrachlorkohlenstoff, 24 ml Triäthylamin und 210 ml Aethylenchlorid wird 3 Stunden unter Rühren und unter Stickstoff bei einer Badtemperatur von 60° gehalten. Anschliessend wird eingedampft, mit Essigester extrahiert und erneut eingedampft. Der Rückstand wird aus Aceton/Hexan umkristallisiert. Man erhält so 10,5 g α-[m-(3,3-Dimethylureido)-phenoxy]-isobutyronitril vom Schmelzpunkt 110°.

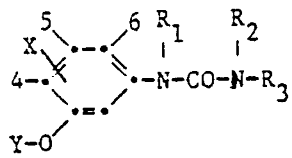

| No | -O-Y | X | $R_1$ | N $R_2$ $R_3$ | physikalische Konstante |
|---|---|---|---|---|---|
| 1 | $OCH_2CN$ | H | H | $N(CH_3)_2$ | Smp. 119-121° |
| 2 | $OCH(CH_3)CN$ | H | H | $N(CH_3)_2$ | Smp. 144-145° |
| 3 | $OCH(CH_3)CN$ | H | H | $N(CH_3)OCH_3$ | Smp. 65° |
| 4 | $OCH_2CN$ | H | H | $N(CH_3)OCH_3$ | $n_D^{30}$ 1.5562 |
| 5 | $OCH_2CH(CH_3)CN$ | H | H | $N(CH_3)_2$ | Smp. 142° |
| 6 | $OCH(C_2H_5)CN$ | H | H | $N(CH_3)_2$ | Smp. 149-150° |
| 7 | $OC(CH_3)_2CN$ | H | H | $N(CH_3)_2$ | Smp. 110° |
| 8 | $OC(CH_3)_2CN$ | H | H | $N(CH_3)OCH_3$ | $n_D^{36}$ 1.5240 |
| 9 | $OCH(CH_3)CN$ | H | H | $N(CH_3)\ C_4H_9\,n$ | Smp. 93-94° |
| 10 | $OCH(CH_3)CN$ | H | H | 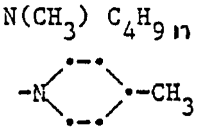 | Smp. 119-120° |
| 11 | $OCH(C_2H_5)CN$ | H | H | $N(CH_3)OCH_3$ | Smp. 95-96° |
| 12 | $OCH(CH_3)CN$ | 4Cl | H | $N(CH_3)_2$ | Smp. 146-147° |
| 13 | $OCH(CH_3)CN$ | H | H | $N(CH_3)CH(CH_3)C\equiv CH$ | |
| 14 | $OCH(C_2H_5)CN$ | H | H | $N(CH_3)CH(CH_3)C\equiv CH$ | |
| 15 | $OCH(C_2H_5)CN$ | H | H | 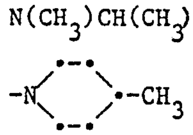 | |
| 16 | $OCH(C_2H_5)CN$ | H | H | $N(CH_3)C_4H_{9n}$ | |
| 17 | $OCH(CN)C_3H_{7n}$ | H | H | $N(CH_3)OCH_3$ | Smp. 83-85° |

| No | -O-Y | X | $R_1$ | N $R_2$ $R_3$ | physikalische Konstante |
|---|---|---|---|---|---|
| 18 | $OCH(CN)C_3H_{7n}$ | H | H | $N(CH_3)_2$ | Smp. 138-140° |
| 19 | $OCH(CN)CH_2OCH_3$ | H | H | $N(CH_3)OCH_3$ | $n_D^{40}$ 1.5323 |
| 20 | $OCH(C_2H_5)CN$ | H | H | $NHCH_3$ | |

Beispiel 3

Das Aufbereiten der Verbindungen der Formel I zu für die Landwirtschaft brauchbaren Anwendungsformen kann beispielsweise gemäss den folgenden Vorschriften erfolgen :

Spritzpulver

Zur Herstellung eines a) 70 %igen und b) 10 %igen Spritzpulvers werden folgende Bestandteile verwendet :

a) 70 Teile α-[m-(3,3-Dimethylureido) phenoxy]-propionitril,
5 Teile Natriumdibutylnaphtylsulfonat,
3 Teile Naphthalinsulfonsäuren-Phenolsulfonsäuren-Formaldehyd-Kondensat 3 : 2 : 1,
10 Teile Kaolin,
12 Teile Champagne-Kreide ;

b) 10 Teile Wirkstoff,
3 Teile Gemisch der Natriumsalze von gesättigten Fettalkoholsulfaten,
5 Teile Naphthalinsulfonsäuren-Formaldehyd-Kondensat,
82 Teile Kaolin.

Der angegebene Wirkstoff wird auf die entsprechenden Trägerstoffe (Kaolin und Kreide) aufgezogen und anschliessend vermischt und vermahlen mit den übrigen Bestandteilen. Man erhält Spritzpulver von vorzüglicher Benetzbarkeit und Schwebefähigkeit. Aus solchen Spritzpulvern können durch Verdünnen mit Wasser Suspensionen von 0,1-8 % Wirkstoff erhalten werden, die sich zur Unkrautbekämpfung in Pflanzenkulturen eignen.

Paste

Zur Herstellung einer 45 %igen Paste werden folgende Stoffe verwendet :

45 Teile α-[m-(3,3-Dimethylureido)-phenoxy]-isobutyronitril,
5 Teile Natriumaluminiumsilikat,
14 Teile Cetylpolyglykoläther mit 8 Mol Aethylenoxid,
1 Teil Oleypolyglykoläther mit 5 Mol Aethylenoxid,
2 Teile Spindelöl,
10 Teile Polyäthylenglykol,
23 Teile Wasser.

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

Emulsionkonzentrat

Zur Herstellung eines 25 %iges Emulsionskonzentrates werden :

25 Teile α-[m-(3,3-Dimethylureido)-phenoxy]-propionitril,
10 Teile einer Mischung von Nonylphenolpolyoxyäthylen oder Calciumdidecylbenzolsulfonat,
10 Teile Cyclohexanon,
55 Teile Xylol,

miteinander vermischt. Dieses Konzentrat kann mit Wasser zu Emulsionen auf geeignete Konzentrationen von z. B. 0,1 % verdünnt werden. Solche Emulsionen eignen sich zur Bekämpfung von Unkräutern in Kulturpflanzungen.

Flowable

Zur Herstellung eines 45 %igen Flowables werden folgende Stoffe verwendet :

45 Teile Aktivsubstanz,
5 Teile Aethylenglycol,
3 Teile Octylphenoxypolyäthylenglycol mit 9-10 Mol Aethylenoxyd pro Mo1 Octylphenol,
3 Teile von einem Gemisch aromatischer Sulfonsulfosäuren, kondensiert mit Formaldehyd als Ammoniumsalz,
1 Teil Siliconöl in Form einer 75 %igen Emulsion,
0,1 Teil einer Mischung von 1-(3-Chlorallyl)-3,5,7-triazoazonium-adamantan-chlorid mit Natriumcarbonat, Chloridwert mind. 11,5 %,
0,2 Teile eines biopolymeren Verdickers mit max. 100 Keimen pro Gramm,
42,7 Teile Wasser.

Die Aktivsubstanz wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

Beispiel 4

Zum Nachweis der Brauchbarkeit als Herbizide (pre- und post-emergent) dienen folgende Testmethoden :

Pre-emergente Herbizid-Wirkung (Keimhemmung)

Im Gewächshaus wird unmittelbar nach der Einsaat der Versuchspflanzen in Saatschalen die Erdoberfläche mit einer wässrigen Dispersion der Wirkstoffe, erhalten aus einem 25 %igen Emulsionskonzentrat resp. aus einem 25 %igen Spritzpulver mit Wirkstoffen, die wegen ungenügender Löslichkeit nicht als Emulsionskonzentrat hergestellt werden können, behandelt. Es wurden verschiedene Konzentrationsreihen angewendet, entsprechend 2 und 1 kg Wirksubstanz pro Hektar. Die Staatschalen werden im Gewächshaus bei 22-25 °C und 50-70 % rel. Luftfeuchtigkeit gehalten und der Versuch nach 3 Wochen ausgewertet und die Resultate nach folgender Notenskala bonitiert :

1 = Pflanzen nicht gekeimt oder total abgestorben,
2-3 = sehr starke Wirkung,
4-6 = mittlere Wirkung,
7-8 = geringe Wirkung,
9 = keine Wirkung (wie unbehandelte Kontrolle).

Post-emergente Herbizid Wirkung (Kontaktherbizid)

Eine grössere Anzahl Unkräuter und Kulturpflanzen, sowohl monocotyle wie dicotyle, wurden nach dem Auflaufen (in 4-bis-6-Blattstadium) mit einer wässrigen Wirkstoffdispersion in Dosierungen von 0,5, 1, 2, und 4 kg Wirksubstanz pro Hektar aud die Pflanzen gespritzt und diese bei 24°-26 °C und 45-60 % relativer Luftfeuchtigkeit gehalten. Mindestens 15 Tage nach der Behandlung wird der Versuch ausgewertet und das Ergebnis wie im pre-emergent-Versuch nach derselben Notenskala bonitiert.

Auch in diesem Versuch zeigten die Verbindungen der Formel I ausgezeichnet Wirkung gegen die breitblättrigen und die meisten der grasartigen Unkräutern, wobei die Kulturpflanze Mais, die Getreidearten Gerste, Hirse und Reis wie auch Baumwolle und Soja nicht oder erst mit höheren Aufwandmenge Wirkstoff geschädigt wurden.

Ergebnisse aus diesen Versuchen sind in den folgenden Tabellen zusammengefasst. Zusätzlich zu den erfindungsgemässen Wirkstoffen wurde N-(3-Chlor-4-methoxyphenyl)-N',N'-dimethylharnstoff (Vergleichssubstanz A) geprüft und nach derselben Notenskala wie vorstehend angegeben bonitiert. Die Vergleichssubstanz A ist aus der französischen Patentschrift Nr. 1.497.868 bekannt.

(Siehe die Tabellen, Seiten 8 und 9)

Vorauflauf

| Verbindung No. | 2 | | 7 | | 8 | | 11 | | A | |
|---|---|---|---|---|---|---|---|---|---|---|
| Aufwandmenge kg/ha | 2 | 1 | 2 | 1 | 2 | 1 | 2 | 1 | 2 | 1 |
| Pflanze | | | | | | | | | | |
| Gerste | 2 | 6 | 1 | 4 | 3 | 4 | 1 | 2 | 2 | 4 |
| Weizen | 2 | 4 | 2 | 3 | 2 | 7 | 1 | 2 | 2 | 3 |
| Mais | 2 | 2 | 4 | 9 | 9 | 9 | 6 | 9 | 3 | 4 |
| Hirse | 7 | 7 | 5 | 7 | 4 | 6 | 4 | 7 | 1 | 2 |
| Reis | 2 | 4 | 4 | 5 | 5 | 5 | 2 | 2 | 1 | 2 |
| avena fatua | 2 | 2 | 1 | 1 | 1 | 3 | 1 | 1 | 1 | 2 |
| lolium perenne | 2 | 4 | 2 | 2 | 1 | 1 | 1 | 1 | 1 | 1 |
| alopecurus myos. | 2 | 2 | 1 | 2 | 1 | 6 | 1 | 2 | 2 | 2 |
| bromus tectorum | 1 | 1 | 1 | 2 | 1 | 2 | 1 | 1 | - | - |
| cyperus rotundus | 8 | 8 | 7 | 8 | 8 | 9 | 7 | 9 | - | - |
| rottboellia exaltata | 2 | 3 | 4 | 8 | 5 | 8 | 9 | 9 | 1 | 2 |
| digitaria sang. | 1 | 1 | 1 | 2 | 4 | 5 | 3 | 9 | 1 | 1 |
| echinochloa crus g. | 2 | 5 | 2 | 4 | 1 | 3 | 1 | 1 | 1 | 1 |
| Soja | 2 | 2 | 2 | 5 | 4 | 8 | 9 | 9 | 1 | 2 |
| Baumwolle | 8 | 8 | 9 | 9 | 8 | 9 | 3 | 9 | 1 | 6 |
| Zuckerrüben | 2 | 2 | 1 | 1 | 1 | 1 | 1 | 1 | 1 | 1 |
| sida spinosa | 1 | 1 | 1 | 1 | 1 | 1 | 1 | 1 | - | - |
| sesbania exaltata | 1 | 1 | 1 | 1 | 1 | 1 | 1 | 2 | 1 | 1 |
| amarantus retroflexus | 1 | 1 | 1 | 2 | 1 | 1 | 1 | 1 | 1 | 1 |
| sinapis alba | 1 | 2 | 1 | 1 | 1 | 1 | 1 | 1 | 1 | 1 |
| impomoea purpurea | 1 | 2 | 1 | 1 | 1 | 1 | 2 | 2 | 1 | 1 |
| galium aparine | 3 | 4 | 3 | 6 | 2 | 7 | 2 | 3 | 1 | 1 |
| chrysanthemum leucum | 1 | 1 | 1 | 1 | 6 | 8 | 1 | 1 | 2 | 2 |
| abutilon sp. | 1 | 1 | 1 | 1 | 1 | 1 | 1 | 1 | - | - |
| solanum nigrum | 1 | 1 | 1 | 1 | 1 | 1 | 1 | 1 | 1 | 1 |

Nachauflauf

| Verbindung No. | 3 | | 6 | | 7 | | 8 | | 11 | | A | |
|---|---|---|---|---|---|---|---|---|---|---|---|---|
| Aufwandmenge kg/ha | 2 | 1 | 2 | 1 | 2 | 1 | 2 | 1 | 2 | 1 | 2 | 1 |
| Pflanze | | | | | | | | | | | | |
| Gerste | 2 | 5 | 1 | 3 | 2 | 3 | 2 | 2 | 2 | 7 | 5 | 7 |
| Weizen | 2 | 7 | 1 | 2 | 2 | 2 | 2 | 4 | 2 | 3 | 9 | 9 |
| Mais | 7 | 9 | 6 | 9 | 9 | 9 | 9 | 9 | 8 | 9 | 5 | 6 |
| Hirse | 4 | 6 | 7 | 9 | 4 | 7 | 2 | 2 | 9 | 9 | 4 | 9 |
| Reis | 2 | 3 | 1 | 2 | 3 | 4 | 2 | 3 | 1 | 2 | 2 | 2 |
| avena fatua | 2 | 4 | 1 | 1 | 1 | 2 | 1 | 1 | 1 | 2 | 2 | 6 |
| lolium perenne | 1 | 4 | 1 | 2 | 6 | 7 | 1 | 2 | 1 | 8 | 2 | 2 |
| alopecurus myos. | 2 | 6 | 1 | 1 | 3 | 3 | 3 | 4 | 1 | 2 | 4 | 4 |
| bromus tectorum | 2 | 3 | 2 | 4 | 3 | 3 | 2 | 2 | 2 | 7 | - | - |
| cyperus rotundus | 4 | 4 | 3 | 4 | 6 | 7 | 8 | 8 | 4 | 6 | 3 | 4 |
| rottboellia exaltata | 1 | 1 | 6 | 9 | 3 | 7 | 1 | 1 | 9 | 9 | 2 | 8 |
| digitaria sang. | 1 | 1 | 1 | 2 | 2 | 3 | 1 | 1 | 2 | 3 | 3 | 4 |
| echinochloa crus g. | 2 | 2 | 1 | 3 | 3 | 3 | 2 | 2 | 2 | 6 | 1 | 2 |
| Soja | 3 | 4 | 7 | 8 | 1 | 1 | 1 | 1 | 8 | 9 | 2 | 4 |
| Baumwolle | 1 | 1 | 4 | 7 | 6 | 7 | 1 | 2 | 1 | 2 | 2 | 3 |
| Zuckerrüben | 1 | 1 | 1 | 2 | 1 | 2 | 1 | 1 | 1 | 1 | 1 | 2 |
| sida spinosa | 1 | 1 | 2 | 2 | 1 | 1 | 1 | 1 | 1 | 1 | - | - |
| sesbania exaltata | 1 | 1 | 1 | 2 | 1 | 1 | 1 | 1 | 1 | 1 | 1 | 3 |
| amarantus retroflexus | 1 | 1 | 1 | 2 | 1 | 1 | 1 | 1 | 2 | 2 | 6 | 6 |
| sinapis alba | 1 | 1 | 1 | 1 | 1 | 1 | 1 | 1 | 1 | 1 | 1 | 2 |
| ipomoea purpurea | 2 | 2 | 1 | 2 | 1 | 1 | 1 | 1 | 2 | 2 | 1 | 1 |
| galium aparine | 1 | 3 | 7 | 8 | 7 | 7 | 1 | 2 | 1 | 4 | 6 | 6 |
| chrysanthemum leucum | 1 | 1 | 1 | 2 | 2 | 2 | 1 | 2 | 1 | 1 | - | - |
| abutilon sp. | 1 | 1 | 1 | 1 | 1 | 1 | 1 | 1 | 1 | 1 | - | - |
| solanum nigrum | 1 | 1 | 1 | 1 | 1 | 1 | 1 | 1 | 1 | 1 | - | - |

**Ansprüche** (für die Vertragstaaten : BE, CH, DE, FR, GB, IT, NL, SE)

1. Neue meta-Cyanoalkoxy-Phenylharnstoffe der Formel I

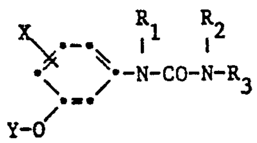

(I)

worin
X Wasserstoff oder Halogen
Y eine Cyanoalkylgruppe

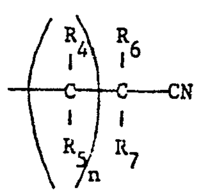

n eine Zahl 0, 1 oder 2
$R_1$ Wasserstoff oder Niederalkyl,
$R_2$ Wasserstoff, Alkyl, Alkenyl, Alkinyl oder Alkoxy,
$R_3$ Wasserstoff, Alkyl oder Alkenyl, oder
$R_2$ und $R_3$ zusammen mit dem Stickstoffatom, an das sie gebunden sind, auch einen 5- bis 6-gliedrigen Heterocyclus, der als Ringglied noch ein Sauerstoffoder Schwefelatom oder eine Iminogruppe enthalten kann,
$R_4$, $R_5$ und $R_7$ unabhängig voneinander je Wasserstoff oder Niederalkyl und
$R_6$ Wasserstoff, Niederalkyl, Niederalkoxy oder Niederalkoxyalkyl bedeuten.

2. Die Verbindungen der Formel I, in denen X Wasserstoff bedeutet.

3. Als Verbindung gemäss Anspruch 1 m-(3,3-Dimethylureido)-phenoxy-acetonitril.

4. Als Verbindung gemäss Anspruch 1 m-(3-Methyl-3-methoxyureido)-phenoxy-acetonitril.

5. Als Verbindung gemäss Anspruch 1 α-[m-(3,3-Dimethylureido)-phenoxy]-propionitril.

6. Als Verbindung gemäss Anspruch 1 α-[m-(3,3-Dimethylureido)-phenoxy]-isobutyronitril.

7. Als Verbindung gemäss Anspruch 1 α-[m-(3-Methyl-3-methoxyureido)-phenoxy]-isobutyronitril.

8. Als Verbindung gemäss Anspruch 1 α-[m-(3-Methyl-3-methoxyureido)-phenoxy]-butyronitril.

9. Verfahren zur Herstellung der Phenylharnstoffe der Formel I, Anspruch 1, dadurch gekennzeichnet, dass man ein entsprechend substituiertes Phenyl der Formel II

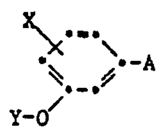

(II)

mit einem Amin der Formel III

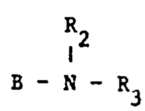

(III)

reagieren lässt, worin $R_2$, $R_3$, X und Y die unter Formel I, Anspruch 1 gegebene Bedeutung haben und eine der beiden Gruppen A und B ein Amin darstellt, während die andere ein Urethan, ein Carbamoylhalogenid, eine Harnstoffgruppe oder den Isocyanatrest darstellt.

10. Verfahren zur Herstellung der Phenylharnstoffe der Formel I, Anspruch 1, dadurch gekennzeichnet, dass man einen meta-Hydroxyphenylharnstoff der Formel IV

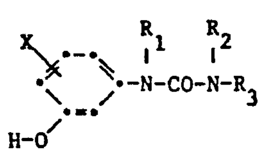

(IV)

in Gegenwart eines säurebindenden Mittels mit einem Cyanoalkylhalogenid der Formel V

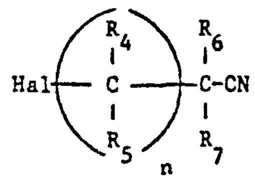

(V)

umsetzt, wobei X, n und $R_1$ bis $R_7$ die unter Formel I, Anspruch 1 gegebene Bedeutung haben.

11. Verfahren zur Herstellung der Phenylharnstoffe der Formel 1, Anspruch 1, dadurch gekennzeichnet, dass man einen in meta-Stellung durch eine Carbamoylalkoxygruppe substituierten Phenylharnstoff der Formel VI

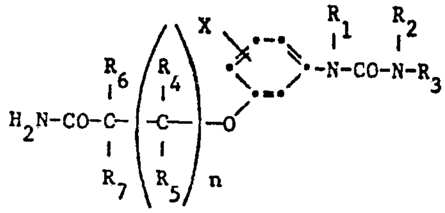

(VI)

worin X, n sowie $R_1$ bis $R_7$ die unter Formel I, Anspruch 1 gegebene Bedeutung haben, mit einem wasserentziehenden Mittel behandelt, bis die Carbamoylalkoxygruppe in die Cyanoalkoxygruppe umgewandelt ist.

12. Herbizides Mittel, dadurch gekennzeichnet, dass es als aktive Komponente mindestens einen meta-Cyanoalkoxy-Phenylharnstoff der Formel I, Anspruch 1 enthält.

13. Die Verwendung der meta-Cyanoalkoxy-Phenylharnstoffe der Formel I, Anspruch 1 zur Bekämpfung unerwünschten Pflanzenwachstums.

14. Die Verwendung gemäss Anspruch 13, der meta-Cyanoalkoxy-Phenylharnstoffe der Formel I, Anspruch 1 zur selektiven Bekämpfung von Unkräutern in Kulturen von Mais, Getreiden, Baumwolle, Soja und Raps.

**Ansprüche** (für den Vertragsstaat AT)

1. Herbizides Mittel, dadurch gekennzeichnet, dass es als aktive Komponente mindestens einen meta-Cyanoalkoxy-Phenylharnstoff der Formel I

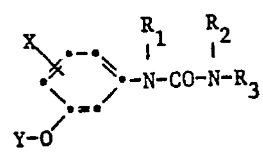

(I)

worin

X Wasserstoff oder Halogen

Y eine Cyanoalkylgruppe

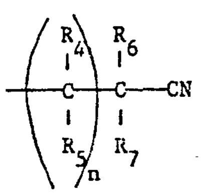

n eine Zahl 0, 1 oder 2

$R_1$ Wasserstoff oder Niederalkyl,
$R_2$ Wasserstoff, Alkyl, Alkenyl, Alkinyl oder Alkoxy,
$R_3$ Wasserstoff, Alkyl oder Alkenyl, oder
$R_2$ und $R_3$ zusammen mit dem Stickstoffatom, an das sie gebunden sind, auch einen 5- bis 6-gliedrigen Heterocyclus, der als Ringglied noch ein Sauerstoff- oder Schwefelatom oder eine Iminogruppe enthalten kann,
$R_4$, $R_5$ und $R_7$ unabhängig voneinander je Wasserstoff oder Niederalkyl und
$R_6$ Wasserstoff, Niederalkyl, Niederalkoxy oder Niederalkoxyalkyl bedeuten, enthält.

2. Mittel nach Anspruch 1, dadurch gekennzeichnet, dass es eine Verbindung der Formel I enthält, in der X Wasserstoff bedeutet

3. Mittel nach Anspruch 1, dadurch gekennzeichnet, dass es m-(3,3-Dimethylureido)-phenoxy-acetonitril enthält.

4. Mittel nach Anspruch 1, dadurch gekennzeichnet, dass es m-(3-Methyl-3-methoxyureido)-phenoxy-acetonitril enthält.

5. Mittel nach Anspruch 1, dadurch gekennzeichnet, dass es α-[m-(3,3-Dimethylureido)-phenoxy]-propionitril enthält.

6. Mittel nach Anspruch 1, dadurch gekennzeichnet, dass es α-[m-(3,3-Dimethylureido)-phenoxy]-isobutyronitril enthält.

7. Mittel nach Anspruch 1, dadurch gekennzeichnet, dass es α-[m-(3-Methyl-3-methoxyureido)-phenoxy]-isobutyronitril enthält.

8. Mittel nach Anspruch 1, dadurch gekennzeichnet, dass es α-[m-(3-Methyl-3-methoxyureido)-phenoxy]-butyronitril enthält.

9. Verfahren zur Herstellung der im Anspruch 1 definierten meta-Cyanoalkoxy-Phenylharnstoffe der Formel I, dadurch gekennzeichnet, dass man ein entsprechend substituiertes Phenyl der Formel II

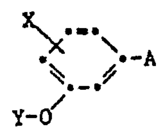

(II)

mit einem Amin der Formel III

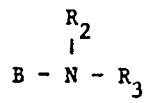

(III)

reagieren lässt, worin $R_2$, $R_3$, X und Y die unter Formel I, Anspruch 1 gegebene Bedeutung haben und eine der beiden Gruppen A und B ein Amin darstellt, während die andere ein Urethan, ein Carbamoylhalogenid, eine Harnstoffgruppe oder den Isocyanatrest darstellt.

10. Verfahren zur Herstellung der im Anspruch 1 definierten meta-Cyanoalkoxy-Phenylharnstoffe der Formel I, dadurch gekennzeichnet, dass man einen meta-Hydroxyphenylharnstoff der Formel IV

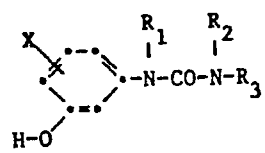

(IV)

in Gegenwart eines säurebindenden Mittels mit einem Cyanoalkylhalogenid der Formel V

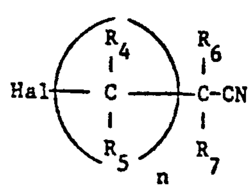

(V)

umsetzt, wobei X, n und $R_1$ bis $R_7$ die unter Formel I, Anspruch 1 gegebene Bedeutung haben.

11. Verfahren zur Herstellung der im Anspruch 1 definierten meta-Cyanoalkoxy-Phenylharnstoffe der Formel I, dadurch gekennzeichnet, dass man einen in meta-Stellung durch eine Carbamoylalkoxygruppe substituierten Phenylharnstoff der Formel VI

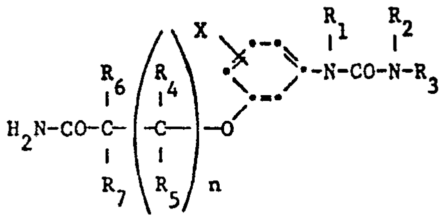

(VI)

worin X, n sowie $R_1$ bis $R_7$ die unter Formel I, Anspruch 1 gegebene Bedeutung haben, mit einem wasserentziehenden Mittel behandelt, bis die Carbamoylalkoxygruppe in die Cyanoalkoxygruppe umgewandelt ist.

12. Die Verwendung der im Anspruch 1 definierten meta-Cyanoalkoxy-Phenylharnstoffe der Formel I zur Bekämpfung unerwünschten Pflanzenwachstums.

13. Die Verwendung gemäss Anspruch 12 der im Anspruch 1 definierten meta-Cyanoalkoxy-Phenylharnstoffe der Formel I zur selektiven Bekämpfung von Unkräutern in Kulturen von Mais, Getreiden, Baumwolle, Soja und Raps.

**Claims** (for the Contracting States : BE, CH, DE, FR, GB, IT, NL, SE)

1. A novel meta-cyanoalkoxy-phenylurea of the formula I

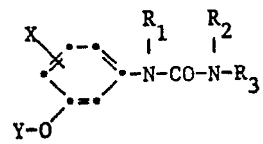

(I)

wherein

X is hydrogen or halogen,

Y is a cyanoalkyl group

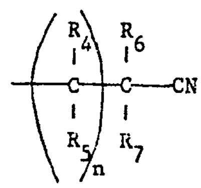

n is a number 0, 1 or 2,

$R_1$ is hydrogen or lower alkyl,

$R_2$ is hydrogen, alkyl, alkenyl, alkynyl or alkoxy,

$R_3$ is hydrogen, alkyl or alkenyl, or

$R_2$ and $R_3$ together with the nitrogen atom to which they are bound form a 5- or 6-membered heterocyclic compound which can also contain as a ring member an oxygen atom or a sulfur atom or an imino group,

$R_4$, $R_5$ and $R_7$ independently of one another are each hydrogen or lower alkyl, and

$R_6$ is hydrogen, lower alkyl, lower alkoxy or lower alkoxyalkyl.

2. A compound of the formula I in which X is hydrogen.

3. m-(3,3-Dimethylureido)-phenoxy-acetonitrile as a compound according to Claim 1.

4. m-(3-Methyl-3-methoxyureido)-phenoxy-acetonitrile as a compound according to Claim 1.

5. α-[m-(3,3-Dimethylureido)-phenoxy]-propionitrile as a compound according to Claim 1.

6. α-[m-(3,3-Dimethylureido)-phenoxy]-isobutyronitrile as a compound according to Claim 1.

7. α-[m-(3-Methyl-3-methoxyureido)-phenoxy]-isobutyronitrile as a compound according to Claim 1.

8. α-[m-(3-Methyl-3-methoxyureido)-phenoxy]-butyronitrile as a compound according to Claim 1.

9. A process for producing a phenylurea of the formula I, Claim 1, which comprises reacting a correspondingly substituted phenyl of the formula II

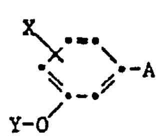

(II)

with an amine of the formula III

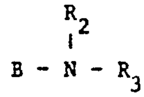

(III)

wherein $R_2$, $R_3$, X and Y have the meanings defined under the formula I, Claim 1, and one of the two groups A and B is an amine, while the other is a urethane, a carbamoyl halide, a urea group or the isocyanate group.

10. A process for producing a phenylurea of the formula I, Claim 1, which comprises reacting a meta-hydroxyphenylurea of the formula IV

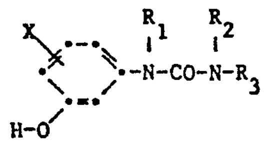

(IV)

in the presence of an acid-binding agent, whith a cyanoalkyl halide of the formula V

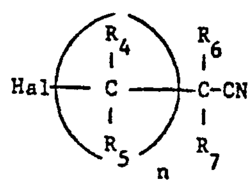

(V)

wherein X, n and $R_1$ to $R_7$ have the meanings given under the formula I, Claim 1.

11. A process for producing a phenylurea of the formula I, Claim 1, which comprises treating a phenylurea of the formula VI which is substituted in the meta-position by a carbamoylalkoxy group

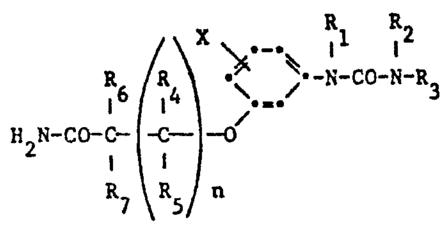

(VI)

wherein X, n and $R_1$ to $R_7$ have the meanings defined under the formula I, Claim 1, with a dehydrating agent until the carbamylalkoxy group has been converted into the cyanoalkoxy group.

12. A herbicidal composition which contains as active ingredient at least one meta-cyanoalkoxy-phenylurea of the formula I, Claim 1.

13. The use of a meta-cyanoalkoxy-phenylurea of the formula I, Claim 1, for controlling undesirable plant growth.

14. The use according to Claim 13 of a meta-cyanoalkoxyphenylurea of the formula I, Claim 1, for the selective combating of weeds in crops of maize, cereals, cotton, soya-bean and rape.

**Claims** (for the Contracting State AT)

1. A herbicidal composition containing as active ingredient at least one meta-cyanoalkoxy-phenylurea of the formula I

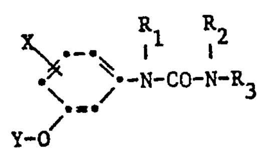

(I)

wherein

X is hydrogen or halogen,

Y is a cyanoalkyl group

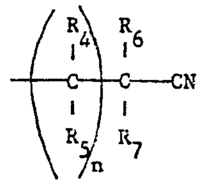

n is a number 0, 1 or 2,
$R_1$ is hydrogen or lower alkyl,
$R_2$ is hydrogen, alkyl, alkenyl, alkynyl or alkoxy,
$R_3$ is hydrogen, alkyl or alkenyl, or
$R_2$ and $R_3$ together with the nitrogen atom to which they are bound form a 5- or 6-membered heterocyclic compound which can contain, as a ring member, also an oxygen or sulfur atom or an imino group,
$R_4$, $R_5$ and $R_7$ independently of one another are each hydrogen or lower alkyl, and
$R_6$ is hydrogen, lower alkyl, lower alkoxy or lower alkoxyalkyl.

2. A composition according to Claim 1, which contains a compound of the formula I wherein X is hydrogen.

3. A composition according to Claim 1, which contains m-(3,3-dimethylureido)-phenoxy-acetonitrile.

4. A composition according to Claim 1, which contains m-(3-methyl-3-methoxyureido)-phenoxy-acetonitrile.

5. A composition according to Claim 1, which contains α-[m-(3,3-dimethylureido)-phenoxy]-propionitrile.

6. A composition according to Claim 1, which contains α-[m-(3,3-dimethylureido)-phenoxy]-isobutyronitrile.

7. A composition according to Claim 1, which contains α-[m-(3-methyl-3-methoxyureido)-phenoxy]-isobutyronitrile.

8. A composition according to Claim 1, which contains α-[m-(3-methyl-3-methoxyureido)-phenoxy]-butyronitrile.

9. A process for producing a meta-cyanoalkoxy-phenylurea of the formula I defined in Claim 1, which comprises reacting a correspondingly substituted phenyl of the formula II

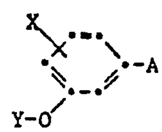

(II)

with an amine of the formula III

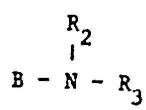

(III)

wherein $R_2$, $R_3$, X and Y have the meanings defined under the formula I, Claim 1, and one of the two groups A and B is an amine, while the other is a urethane, a carbamoyl halide, a urea group or the isocyanate group.

10. A process for producing a meta-cyanoalkoxy-phenylurea of the formula I, which comprises reacting a meta-hydroxyphenylurea of the formula IV

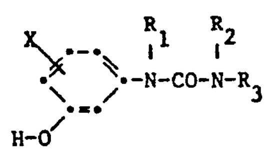

(IV)

in the presence of an acid-binding agent, with a cyanoalkyl halide of the formula V

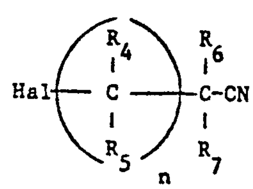

(V)

wherein X, n and $R_1$ to $R_7$ have the meanings defined under the formula I, Claim 1.

11. A process for producing a meta-cyanoalkoxy-phenylurea of the formula I defined in Claim 1, which comprises treating a phenylurea of the formula VI which is substituted in the meta-position by a carbamoylalkoxy group

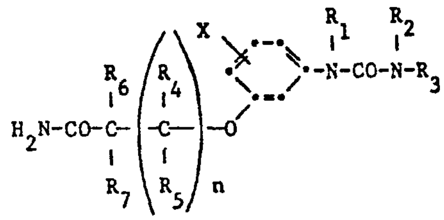

(VI)

wherein X, n and $R_1$ to $R_7$ have the meanings defined under the formula I, Claim 1, with a dehydrating agent until the carbamoylalkoxy group has been converted into the cyanoalkoxy group.

12. The use of a meta-cyanoalkoxy-phenylurea of the formula I defined in Claim 1 for combating undesirable plant growth.

13. The use according to Claim 12 of a meta-cyanoalkoxyphenylurea of the formula I defined in Claim 1 for the selective combating of weeds in crops of maize, cereals, cotton, soya-bean and rape.

**Revendications** (pour les Etats contractants : BE, CH, DE, FR, GB, IT, NL, SE)

1. Nouvelles méta-cyanalcoxy-phénylurées de formule I

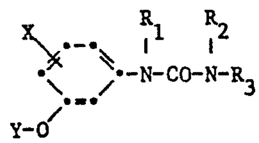

(I)

dans laquelle

X représente l'hydrogène ou un halogène,

Y représente un groupe cyanalkyle

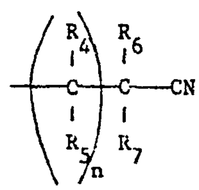

n est égal à 0, 1 ou 2,

$R_1$ représente l'hydrogène, un groupe alkyle inférieur,

$R_2$ représente l'hydrogène, un groupe alkyle, alcényle, alcynyle ou alcoxy,

$R_3$ représente l'hydrogène, un groupe alkyle ou alcényle, ou bien

$R_2$ et $R_3$ forment ensemble et avec l'atome d'azote sur lequel ils sont fixés un hétérocycle à 5 ou 6 chaînons qui peut encore contenir en tant que chaînon cyclique un atome d'oxygène ou de soufre ou un groupe imino,

$R_4$, $R_5$ et $R_7$ représentent chacun, indépendamment les uns des autres, l'hydrogène ou un groupe alkyle inférieur, et

$R_6$ représente l'hydrogène, un groupe alkyle inférieur, alcoxy inférieur ou alcoxyalkyle inférieur.

2. Les composés de formule I dans laquelle X représente l'hydrogène.

3. En tant que composé selon la revendication 1, le m-(3,3-diméthyluréido)-phénoxy-acétonitrile.

4. En tant que composé selon la revendication 1, le m-(3-méthyl-3-méthoxy-uréido)-phénoxy-acétonitrile.

5. En tant que composé selon la revendication 1, l'α-[m-(3,3-diméthyluréido)-phénoxy]-propionitrile..

6. En tant que composé selon la revendication 1, l'α-[m-(3,3-diméthyluréido)-phénoxy]-isobutyronitrile.

7. En tant que composé selon la revendication 1, l'α-[m-(3-méthyl-3-méthoxy-uréido)-phénoxy]-isobutyronitrile.

8. En tant que composé selon la revendication 1, l'α-[m-(3-méthyl-3-méthoxy-uréido)-phénoxy]-butyronitrile.

9. Procédé de préparation des phénylurées de formule I, selon la revendication 1, caractérisé en ce que l'on fait réagir un dérivé phénylique portant les substituants correspondants et répondant à la formule II

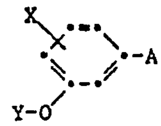

(II)

avec une amine de formule III

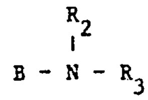

(III)

$R_2$, $R_3$, X et Y ayant les significations indiquées en référence à la formule I, selon la revendication 1, et l'un des deux groupes A et B représentant une amine alors que l'autre représente un uréthane, un halogénure de carbamoyle, un groupe urée ou le reste isocyanate.

10. Procédé de préparation des phénylurées de formule 1, selon la revendication 1, caractérisé en ce que l'on fait réagir une méta-hydroxyphénylurée de formule IV :

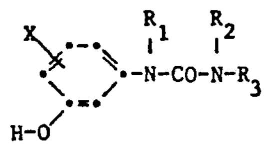

(IV)

en présence d'un agent fixant les acides, avec un halogénure de cyanalkyle de formule V :

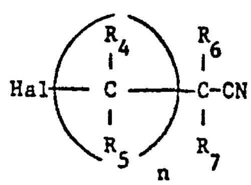

(V)

X, n et $R_1$ à $R_7$ ayant les significations indiquées en référence à la formule I, selon la revendication 1.

11. Procédé de préparation des phénylurées de formule I, selon la revendication 1, caractérisé en ce que l'on traite une phénylurée substituée en position méta par un groupe carbamoylalcoxy et répondant à la formule VI :

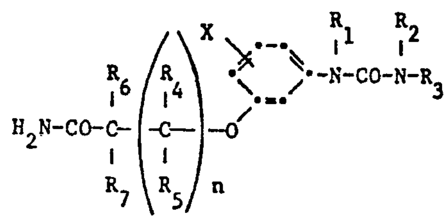

(VI)

dans laquelle X, n et $R_1$ à $R_7$ ont les significations indiquées en référence à la formule I, selon la revendication 1, par un agent déshydratant, jusqu'à conversion du groupe carbamoylalcoxy en le groupe cyanalcoxy.

12. Produit herbicide caractérisé en ce qu'il contient en tant que composant actif au moins une métacyanalcoxy-phénylurée de formule I, selon la revendication 1.

13. L'utilisation des méta-cyanalcoxy-phénylurées de formule I, selon la revendication 1, dans la lutte contre les croissances des végétaux indésirables.

14. L'utilisation selon la revendication 13 des méta-cyanalcoxy-phénylurées de formule I, selon la revendication 1, dans la lutte sélective contre les mauvaises herbes dans les cultures de maïs, de céréales, de coton, de soja et de colza.

**Revendications** (pour l'Etat contractant AT)

1. Produit herbicide caractérisé en ce qu'il contient en tant que composant actif au moins une méta-cyanalcoxy-phénylurée de formule I

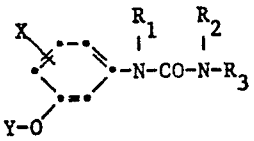

(I)

dans laquelle

X représente l'hydrogène ou un halogène,

Y représente un groupe cyanalkyle

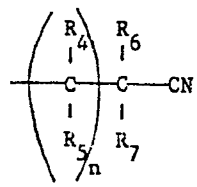

n est égal à 0,1 ou 2,

$R_1$ représente l'hydrogène, un groupe alkyle inférieur,

$R_2$ représente l'hydrogène, un groupe alkyle, alcényle, alcynyle ou alcoxy,

$R_3$ représente l'hydrogène, un groupe alkyle ou alcényle, ou bien

$R_2$ et $R_3$ forment ensemble et avec l'atome d'azote sur lequel ils sont fixés un hétérocycle à 5 ou 6 chaînons qui peut encore contenir en tant que chaînon cyclique un atome d'oxygène ou de soufre ou un groupe imino,

$R_4$, $R_5$ et $R_7$ représentent chacun, indépendamment les uns des autres, l'hydrogène ou un groupe alkyle inférieur, et

$R_6$ représente l'hydrogène, un groupe alkyle inférieur, alcoxy inférieur ou alcoxyalkyle inférieur.

2. Produit selon la revendication 1, caractérisé en ce qu'il contient un composé de formule I, dans laquelle X représente l'hydrogène.

3. Produit selon la revendication 1, caractérisé en ce qu'il contient le m-(3,3-diméthyluréido)-phénoxy-acétonitrile.

4. Produit selon la revendication 1, caractérisé en ce qu'il contient le m-(3-méthyl-3-méthoxy-uréido)-phénoxy-acétonitrile.

5. Produit selon la revendication 1, caractérisé en ce qu'il contient l'α-[m-(3,3-diméthyluréido)-phénoxy]-propionitrile.

6. Produit selon la revendication 1, caractérisé en ce qu'il contient l'α-[m-(3,3-diméthyluréido)-phénoxy]-isobutyronitrile.

7. Produit selon la revendication 1, caractérisé en ce qu'il contient l'α-[m-(3-méthyl-3-méthoxy-uréido)-phénoxy]-isobutyronitrile.

8. Produit selon la revendication 1, caractérisé en ce qu'il contient l'α-[m-(3-méthyl-3-méthoxy-uréido)-phénoxy]-butyronitrile.

9. Procédé de préparation des méta-cyanalcoxy-phénylurées de formule I définie dans la revendication 1, caractérisé en ce que l'on fait réagir un dérivé phénylique portant les substituants correspondants et répondant à la formule II :

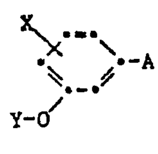

(II)

avec une amine de formule III

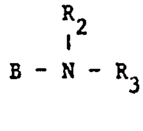

(III)

$R_2$, $R_3$, X et Y ayant les significations indiquées en référence à la formule I, selon la revendication 1, et l'un des deux groupes A et B représentant une amine alors que l'autre représente un uréthane, un halogénure de carbamoyle, un groupe urée ou le reste isocyanate.

10. Procédé de préparation des méta-cyanalcoxyphénylurées de formule I définie dans la revendication 1, caractérisé en ce que l'on fait réagir une méta-hydroxyphénylurée de formule IV

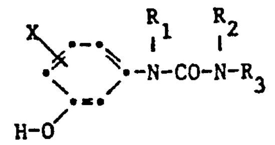

(IV)

en présence d'un agent fixant les acides, avec un halogénure de cyanalkyle de formule V

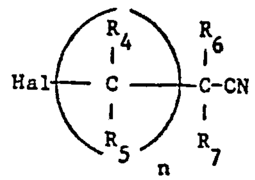

(V)

X, n et $R_1$ à $R_7$ ayant les significations indiquées en référence à la formule I, selon la revendication 1.

11. Procédé de préparation des méta-cyanalcoxyphénylurées de formule I définie dans la revendication 1, caractérisé en ce que l'on traite une phénylurée substituée en position méta par un groupe carbamoylalcoxy et répondant à la formule VI :

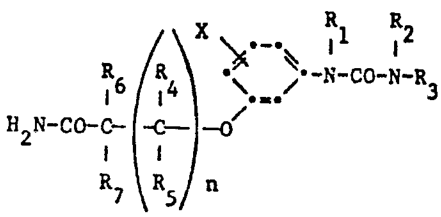

(VI)

dans laquelle X, n et $R_1$ à $R_7$ ont les significations indiquées en référence à la formule I, selon la revendication 1, par un agent déshydratant, jusqu'à conversion du groupe carbamoylalcoxy en le groupe cyanalcoxy.

12. L'utilisation des méta-cyanalcoxy-phénylurées de formule I, définie dans la revendication 1, dans la lutte contre les croissances des végétaux indésirables.

13. L'utilisation selon la revendication 12 des méta-cyanalcoxy-phénylurées de formule I, définie dans la revendication 1, dans la lutte sélective contre les mauvaises herbes dans les cultures de maïs, de céréales, de coton, de soja et de colza.